# EUROPEAN PATENT APPLICATION

(11) **EP 3 311 881 A1**
(43) Date of publication of application: **25.04.2018**
(21) Application number: 17197045.2
(22) Date of filing: 18.10.2017
(51) Int. Cl.: A61N 1/362, A61N 1/368, A61N 1/37

(54) **APPARATUS AND METHOD TO OPTIMIZE PACING PARAMETERS**

(30) Priority: 20.10.2016 US 201662410417 P
(71) Applicant: BIOTRONIK SE & Co. KG, 12359 Berlin (DE)
(72) Inventor: Taff, Brian M., Portland, OR Oregon 97217 (US)
(74) Representative: Keck, Hans-Georg

(57) **Abstract**

The invention refers to an implantable medical device (100) comprising a control unit (62), at least a right ventricular sensing unit (58) and/or a right atrial sensing unit, wherein the right ventricular sensing unit (58) is connected or can be connected to a right ventricular stimulation electrode lead (110) having at least one electrode pole and a stimulation unit (54) that is connected or can be connected to a multipolar left ventricular stimulation electrode lead (114) having a plurality of electrode poles (141, 142, 143, 144). The control unit (62) is adapted to determine right atrioventricular conduction state and to cause selection of one of the plurality of electrode poles (141, 142, 143, 144) of the multipolar left ventricular stimulation electrode lead (114) depending a respective determined right atrioventricular conduction state.

## Description

The invention refers to an implantable medical device comprising a control unit, and at least a right ventricular sensing unit that is connected, or can be connected, to a right ventricular stimulation electrode lead having at least one electrode pole and a stimulation unit that is connected, or can be connected, to a multipolar left ventricular stimulation electrode lead having a plurality of electrode poles.

The invention particularly refers to a heart stimulator intended to administer cardiac resynchronization therapy (CRT). Such a heart stimulator can embody an implantable cardiac pacemaker or similar functionality as enabled via implantable cardiac cardioverter/defibrillator devices.

Cardiac resynchronization therapy (CRT) stands as one of the primary treatments for patients suffering from heart failure (HF). Numerous methods have been proposed and/or developed for enabling improved programming of CRT devices, with a particular focus on the optimization of two particular timing parameters: 1.) the atrio-ventricular delay (AVD) and 2.) the inter-ventricular delay (VVD). The invention herein, further explores device feature support as a means for improving patient response to CRT.

Implantable heart stimulators can be used for cardiac rhythm management (CRM) for treating a variety of heart functional and rhythm disorders by way of electric stimulation pulses delivered to the heart tissue (i.e. the myocardium). A sufficiently strong stimulation pulse administered outside of a heart chamber's refractory period leads to excitation of the myocardium, facilitating a contraction of the respective heart chamber.

Based upon the therapeutic need, such a heart stimulator delivers electrical stimulation pulses to the heart tissue (myocardium) in accordance with a relevant, prescriptive timing regimen. Administration of these stimulation pulses is generally achieved using lead-based electrodes that interface with the patient myocardium and connect said tissue to stimulation pulse generators located within the heart stimulator's housing. In the context of this architecture, a stimulation pulse is referred to as a pace. Delivering these paces to individual heart chambers, in turn, facilitates the coordinated electrical stimulation of heart activity.

To effectively sense the natural contraction of a heart chamber that occurs without artificial stimulation, a dynamic referred to as intrinsic contraction, the heart stimulator usually includes at least one sensing stage that is connected to a sensing electrode and said electrode is typically placed in or near the heart chamber. An intrinsic excitation of a heart chamber results in characteristic electrical potentials that can be picked up via the sensing electrode and that can be evaluated by the sensing stage to determine whether an intrinsic excitation - called: intrinsic event - has occurred.

Usually, a heart stimulator features separate stimulation pulse generators for each heart chamber intended to be stimulated. Therefore, in a dual chamber pacemaker, usually an atrial and a ventricular stimulation pulse generator are provided for generating atrial and ventricular stimulation pulses. Delivery of an atrial or a ventricular stimulation pulse causing an artificial excitation of the atrium or the ventricle, respectively, is called an atrial stimulation event AP (atrial paced event) or a ventricular stimulation event VP (ventricular paced event), respectively.

Similarly, common heart stimulators feature separate sensing stages for each heart chamber of interest. In a dual chamber pacemaker usually two separate sensing stages, an atrial sensing stage and a ventricular sensing stage, are provided that are capable of detecting intrinsic atrial events AS (atrial sensed event) or intrinsic ventricular events VS (ventricular sensed event), respectively.

In a heart cycle, an excitation of the myocardium leads to a depolarization of the myocardium that leads to a contraction of the heart chamber. If the myocardium is fully depolarized it is unsusceptible to further excitation and is thus refractory. Thereafter, the myocardium repolarizes and thus relaxes and the heart chamber expands again. In a typical intracardiac electrogram (IEGM), depolarization of the ventricle corresponds to a signal known as the "R-wave". The repolarization of the ventricular myocardium coincides with a signal known as the "T-wave". Atrial depolarization is manifested by a signal known as the "P-wave".

In a healthy heart, initiation of the cardiac cycle normally begins with depolarization of the sinoatrial (SA) node. This specialized structure is located in the upper portion of the right atrium wall and acts as a natural "pacemaker" of the heart. In a normal cardiac cycle and in response to the initiating SA depolarization, the right atrium contracts and forces the blood that has accumulated therein into the ventricle. The natural stimulus causing the right atrium to contract is conducted to the right ventricle via the atrioventricular node (AV node) with a short, natural delay, known as the atrioventricular delay (AV-delay). Thus, a short time after the right atrial contraction (a time sufficient to allow the bulk of the blood in the right atrium to flow through the one-way valve into the right ventricle), the right ventricle contracts, forcing the blood out of the right ventricle to the patient's lungs for reoxigenation. A typical time interval between contraction of the right atrium and contraction of the right ventricle might be 100 - 150 ms; a typical time interval between contraction of the right ventricle and the next contraction of the right atrium might be 800 ms. Thus, it is a right atrial contraction (A), followed a relatively short time thereafter by a right ventricle contraction (V) that produces the desired AV synchrony, followed a relatively long time thereafter by the next right atrial contraction, that produces the desired cardiac cycle timing in the patient/device interaction dynamic. Where AV synchrony exists, the heart functions more efficiently as a pump for circulating blood within the patient's anatomy; where AV synchrony is absent, the heart functions as an inefficient pump (largely because the right ventricle is contracting when it is not filled with blood). Similarly, in a healthy heart, the left ventricle ideally contracts in synchrony with atrial activity and also in coordination with the right ventricle to facilitate various modes of biventricular pacing support.

A pacemaker generally induces a contraction of a heart chamber by delivery of a stimulation pulse (pacing pulse) to said chamber when natural (intrinsic) contraction of said chamber fails to occurs in due time. A contraction of a heart chamber is commonly branded as an "event". Contractions may be intrinsic and termed sensed events or result from the delivery of a stimulation pulse and, in turn, termed paced events. As such, a sensed event in the atrium is referred to as an As, while a paced atrial event is referred to as an Ap. Similarly, a sensed event in the ventricle is branded as a Vs and a paced ventricular event is branded as a Vp.

To mimic the natural behavior of a heart, a dual-chamber pacemaker employs an AV-delay timer to provide an adequate time delay (atrioventricular delay, AV-delay, AVD) between a natural (intrinsic), stimulated (paced), or scheduled (phantom) right atrial event and a right ventricular contraction. Since an optimal AV-delay may vary for different heart rates or stimulation rates, and may even vary from patient to patient, the AV-delay is typically adjustable with ranges regularly spanning from 10s of ms to upwards of 500 ms. In a similar way, a biventricular pacemaker extends this support by slightly redefining the AVD as the delay between an atrial event and the first-paced ventricular chamber, and subsequently provisioning a VV-delay (VVD) timer to coordinate pacing in both ventricles. The VVD timer initiates at the termination of the AVD and, in turn, pacing nominally occurs in the last-paced chamber at the expiration of the VVD timer. The VVD, can be programmed, with ranges that typically span from 0 ms ... +100 ms. Herein the 0 ms condition would represent the simultaneous pacing of both ventricles while increased VVD values would dictate when the second ventricle (either RV or LV) would be scheduled to follow the first-paced chamber. In triple-chamber devices, a left ventricular (stimulated, paced) contraction may be associated with either right ventricular events or right atrial events. The former case caters to RV-first biventricular pacing and LV-only pacing support modalities where either RV pacing occurs at the expiration of the AVD and LV pacing is administered at the end of the AVD+VVD duration or RVs events start VVD timers that coordinate the LV-only output. Alternatively, in LV-first biventricular pacing support, LV paces are administered at the expiration of the AVD and the affiliate RV pacing support is administered at the end of the AVD+VVD duration.

To deal with possibly occurring natural (intrinsic) atrial or ventricular contractions, a demand pacemaker schedules a stimulation pulse for delivery at the end of the AV-delay or the VV-delay, respectively. The delivery of said stimulation pulse is inhibited if a natural contraction is sensed within the respective time delay in the chamber intended for stimulation. (In fact, certain algorithms available within current state-of-the art pacing systems such as AV hysteresis are often used to promote this type of intrinsic activity as a means for encouraging the patient's baseline physiology to support patient needs in ways that prolong implant service times and may more effectively combat the progression of key disease states.) A natural contraction of a heart chamber can be detected by evaluating electrical signals sensed by the sensing channels. In the sensed electrical signal the depolarization of the atrial tissue is manifested by occurrence of a P-wave. Similarly, the depolarization of ventricular tissue is manifested by the occurrence of an R-wave. The detection of a P-wave or an R-wave signifies the occurrence of intrinsic atrial, As, or ventricular, Vs events, respectively.

A dual chamber pacemaker featuring an atrial and a ventricular sensing stage and an atrial and a ventricular stimulation pulse generator can be operated in a number of stimulation modes. In VVI modes, for example, atrial sense events are ignored and no atrial stimulation pulses are generated, but ventricular stimulation pulses are delivered in a demand capacity. Conversely, in AAI modes ventricular sense events are ignored and no ventricular stimulation pulses are generated, but atrial stimulation pulses are delivered in a demand capacity. Further, in DDD modes, both atrial and ventricular stimulation pulses are delivered in a demand capacity and ventricular stimulation pulses can be generated in synchrony with sensed or paced atrial events, wherein ventricular stimulation pulses follow atrial activity subject to an appropriate atrioventricular delay (AV-delay; AVD). Such device/patient interactions maintain the hemodynamic benefit of optimized pump behavior. As a general remark, VVI, AAI, and DDD modes represent distinct classes of mode support, but are not the sole configurations supported by state of the art implants and, as such, the description herein by no means intends to limit the paired mode support enabled by the present invention.

For patients with Heart Failure (HF), biventricular pacing, as facilitated by triple chamber pacing devices, offers critical clinical benefit. This alignment between patient indication and medical device support stems from the ability of triple-chamber pacing systems to enable Cardiac Resynchronization Therapies (CRT) that combat the ventricular dysfunction emergent from, among other considerations, ventricular remodeling in HF patients. (Both CRT-P (CRT with pacemaker function) and CRT-D (CRT with pacemaker and ICD function) devices enable therapeutic support of this type.) The ventricular remodeling processes linked to HF causes ventricular geometry changes that drive affiliate conduction system abnormalities, inclusive of sub-optimal inter- and intra-ventricular conduction delays. Such circumstances create ventricular dyssynchrony where contraction in the ventricles no longer coordinates to enable efficient cardiac output. Depending upon the patient's baseline conduction status, CRT therapies may be adapted to employ pacing in both ventricles or leverage variants that offer RV or LV-only stimulus. By electrically stimulating the site of latest ventricular activation, CRT, in turn, forces the right and left ventricle to contract in a more coordinated manner. As such, CRT promotes a more synchronized pumping of the ventricles, increasing the efficiency of ventricular contraction and improving the patient's affiliate hemodynamics.

Despite these noted therapeutic gains, clinical experience has shown that nearly one third of CRT candidates fail to respond to the therapy as intended. Possible reasons for the prevalence of this patient sub-population may include inappropriate/sub-optimal coronary sinus (CS) lead positioning (the typical placement for LV leads), regional ventricular infarctions, and pacing parameter settings that are poorly tuned to the needs of the individual, among others. Against this clinical backdrop, evidence has shown that optimizing pacing parameters, particularly the AVD and VVD, offers the potential to change some CRT non-respondents to respondents. Even for the respondents, AVD and VVD optimization typically stand to improve the efficacy of the administered CRT.

CRT optimization can be accomplished using echo techniques inclusive of Doppler imaging. CRT optimization can also be achieved on the basis of metrics derived from the ECG or EGM signal, such as the P-wave duration and/or QRS duration. Alternatively, the device settings can be optimized based on hemodynamic indices that are assumed to correlate to the stroke volume or cardiac output, such as the blood pressure or its temporal derivative (typically tuning to maximize LV dP/dt), the blood oxygen saturation, blood pH, etc. In addition, CRT optimization has been further proposed by means of intracardiac impedance measurement feedback mechanics which provide a surrogate means for assessing ventricular volume.

AVD optimization, which aims to maintain synchronized timing between the atria and ventricles, has been a focus of research since the early development of the dual-chamber pacemakers. The introduction of CRT in the late 1990s generated further interests in AVD optimization and brought on a new challenge for VVD optimization, which aims to coordinate RV and LV contractions. Moreover, AVD and VVD optimizations have been further complicated by additional confounding factors, which include their variations subject to the pacing rate and the pacing mode. Given this complexity, numerous methods have been proposed and/or developed for optimal programming of the AVD and VVD in CRT devices.

For patients with intact sinus rhythm or with effective atrial pacing, it is beneficial to stimulate the ventricle(s) in a manner that is synchronous with the atrial activation, i.e., instated with a prescribed delay following the atrial event. As mentioned prior, standard AV-synchronous dual- or triple-chamber implantable devices offer a user-programmable AVD. Several studies have shown the importance of optimizing the AVD on a patient-specific basis to improve cardiac output. For Chronic Heart Failure (CHF) patients especially, an optimization of the AVD is essential. As the pumping efficacy is impaired, the optimal timing of the ventricular stimulus in relation to the atrial event contributes significantly to cardiac performance. If the AVD is too short, the ventricle contracts before fully filling with blood ejected from the atria. In such contexts, the stroke volume and the cardiac output is reduced. Alternatively, if the AVD is too long, the ventricle contracts well after the closure of the atrioventricular valve causing a concomitant reduction in the potential benefit offered from the atrial kick. Additionally, in cases of excessive AVD, backflow of blood from the ventricle into the atrium, i.e., mitral regurgitation, proves likely, further reducing cardiac output. The optimal AVD furthermore depends on the activation state of the circulation. If the sympathetic tone is high, e.g., during exercise, the optimal AVD is shortened compared to the resting value.

Several methods for individual AVD optimization are state of the art. The adjustment of AVD in most cases is performed during the follow-up procedure by the physician with external measurement systems, not by the implant itself. In most of these methods, the patient resides in a resting condition during the adjustment procedure and therefore only the 'static' AVD is optimized. Although modern pacing devices possess a programmable dynamic AVD, i.e., an AVD that depends on the heart rate, the dynamic values are estimated in the majority of cases.

It is an objective of the invention to provide a heart stimulator comprising a stimulation control unit and one or more stimulation units that can determine beneficial pacing parameters. The invention thus targets a means for addressing patient populations that show no meaningful clinical response to CRT therapies.

Preferably, the implantable medical device comprises a control unit, and, at least, a right ventricular sensing unit and/or a right atrial sensing unit, wherein the right ventricular sensing unit is connected, or can be connected, to a right ventricular stimulation electrode lead having at least one electrode pole and a stimulation unit that is connected, or can be connected, to a left ventricular stimulation electrode lead having a plurality of electrode poles. The control unit is adapted to determine right atrioventricular conduction status and as such, may monitor sensed atrial (likely using a dedicated atrial lead) and/or ventricular activities to motivate and/or provide selection of one or more of the plurality of electrode poles found on the multipolar left ventricular stimulation lead in response to said conduction status. Preferably, the control unit is connected, or can be connected, to a right ventricular stimulation electrode and/or the left ventricular stimulation electrode.

According to an embodiment of the present invention, the control unit is configured to determine whether a right ventricular sense-event (RVs) is present or not for determining right side atrio-ventricular conduction state. Moreover, the control unit is configured to apply one LV pacing vector in case RVs events are lacking, and apply another LV pacing support vector when RVs events are present.

In one embodiment of the present invention, the control unit is adapted to discriminate between at least two right-side atrioventricular conduction states, including a presence of intrinsic right-side atrioventricular conduction and/or an absence of right-side atrioventricular conduction (with possible further sub-classification and management on the basis of specific disturbed right-side atrioventricular conduction states).

In one embodiment of the present invention, the control unit is adapted to identify atrioventricular conduction state according to measurements indicating right ventricular (RV) activity. For example, the control unit can be configured to classify and associate RV activity to the atrioventricular conduction state according to the following scheme:
1. Right ventricular sense-event (RVs) activity is coordinated well with preceding atrial events - corresponds to a viable right side conduction;
2. RVs activity is poorly coordinated with preceding atrial events - corresponds to a slow atrioventricular conduction or the like;
3. the presence of paced right ventricular events (RVp) - corresponds to a complete lack of conduction on the right side of the heart.

Preferably, the control unit is adapted to determine the presence or absence of an intrinsic right-side atrioventricular conduction depending on a temporal condition based on right-side ventricular activity with a preceding intrinsic or stimulated right atrial event (As; Ap).

According to an embodiment of the present invention, the control unit is adapted to determine the presence of an intrinsic right-side atrioventricular conduction if a right ventricular sense-event (RVs) is recorded within a predetermined time period starting from an immediately preceding intrinsic or a stimulated right atrial event (As; Ap).

According to an embodiment of the present invention, the implantable medical device is configured to assess the right-side conduction state in a periodical manner, for instance on a daily basis. An assessment of the right-side atrioventricular conduction state may comprise several consecutive measurements. For example, each measurement may take one minute, wherein a measured right-side conduction state or a change of that state may be confirmed as soon as a predefined number of consecutive measurements show the same result. The LV pacing vector can be chosen and updated according to said periodical assessments of the right-side atrioventricular state. Moreover, said periodical assessments may be entirely independent of any automated or periodic checks of the interventricular conduction status (e.g. RV to LV).

The lack of adaptation in pacing site selection based upon right atrioventricular conducted responses (the norm for state of the art devices), can compromise the efficacy of biventricular (BiV) pacing such that even high percentage BiV support (a typical hallmark of improved CRT response) may not promote coordinated ventricular activities that best serve the patient. The clinician might then take added measures (drug administration, device parameter setting changes, etc.) that prove entirely tangential and non-therapeutically beneficial to the patient. Time wasted in the context of heart failure patients can be especially precious since patient health can degrade precipitously.

Left ventricular lead position (which in the context of increasingly prevalent quadri-polar lead options can be reinterpreted as optimal left ventricular pacing vector selection) has been highlighted as a potentially critical reason for such suboptimal response in certain patients. Left ventricular pacing is typically deemed optimal when it is administered at the latest activated region within the left ventricle. The site of latest left ventricular activation has historically been determined in response to intrinsic right atrioventricular conduction activity without additional explicit consideration for latest left ventricular site locations emergent from paced right ventricular events. In many patients, it has been determined that the presence or absence of intrinsic right atrioventricular conduction (A to RV) activity can shift the physical location of the latest activated region within the left ventricle. As such, optimal pacing sites would differ depending upon the patient's prevailing conduction status. The present invention proposes monitoring a patient's intrinsic right atrioventricular conduction status and, in turn, using that status to either 1.) administer left ventricular pacing at the latest activated site subject to intrinsic right ventricular activity when right atrioventricular conduction is present or 2.) administer left ventricular pacing at the latest activated site subject to paced right ventricular events when right atrioventricular conduction is absent.

In the context of the present invention, the terms 'electrode' or 'vector' are used likewise for an electrode and its associated electrical pathway through the heart, for instance in the context of selecting an optimal electrical pathway for stimulation.

In an embodiment of the present invention, specific left ventricular electrodes for left ventricular pacing are linked to different states of right atrioventricular conduction. Exemplary states of right atrioventricular conduction could be:
- Intrinsic right atrioventricular conduction exists, indicated e.g. by measuring right ventricular events that pair with preceding right atrial events;
- intrinsic right atrioventricular conduction is disturbed, wherein that state can be further differentiated into more specific classes according to the type of measured disturbance. For instance, a disturbed state can be indicated by measuring irregular or dissociated right ventricular events with preceding right atrial events, or a missing one-to-one association between a right atrial event and a sensed right ventricular event;
- intrinsic right atrioventricular conduction is missing.

In a preferred embodiment, for each state of right atrioventricular conduction suitable left ventricular electrodes for left ventricular pacing are defined. Preferably, the choices for left ventricular electrodes should at least be dependent on the presence or absence of sensed right ventricular events in the cardiac cycle and the relative timing of any observed sensed right ventricular event with respect to preceding right atrial events. For this purpose, said left ventricular electrodes can be chosen and programmed manually by the physician, for example during a follow up examination. Alternatively, the left ventricular electrodes can be selected and adjusted by the implantable medical device in use. In the latter case, the implantable medical device may determine suitable left ventricular electrodes according to recently sensed events of the right atrioventricular conduction system and with respect to a detected right atrioventricular conduction state. The automated selection process by the implantable device can moreover be subject to various criteria defined by the physician according to thresholds and/or limits. For example, the physician may preset that in case of low battery state of the implantable device, a left ventricular electrode which is highly energy consuming when used for pacing should not be automatically selected. In embodiments of the present invention, the implantable medical device may be configured such that selection and/or adjustment of left ventricular electrodes can be performed both manually and automatically or in a combined manner. In an embodiment of the present invention, the implantable medical device senses interventricular conduction automatically and adjusts suitable left ventricular electrodes in either a continuous or periodical manner, again with reference to the prevailing right-side conduction condition.

For selecting suitable left ventricular electrodes, the implantable medical device may be capable of performing tests for acquiring interventricular conduction times, in particular RV to LV conduction tests. Such tests can be based on iterative
- sensing of the right ventricle events (when intrinsic right atrioventricular conduction exists) and/or
- pacing of the right ventricle (when intrinsic right atrioventricular conduction is missing or overdriven), followed by
- sensing the ensuing event in the left ventricle at different left ventricular electrodes,
- comparing and analyzing the different conduction times between the right ventricular event and the sensed left ventricular event for each left ventricular electrode,
- selecting, in a preferred embodiment of the invention, the left ventricular electrode having the longest conduction time as left ventricular pacing electrode.

According to an embodiment of the present invention, the implantable medical device is configured to check for the presence of RVs events and
- apply one LV pacing vector in case RVs events are lacking, and
- apply another LV pacing support vector when RVs events are present.

The left ventricular electrode determined by said exemplary method may then be assigned to the corresponding right atrioventricular state. In a preferred embodiment, the information on the selected left ventricular electrode and the corresponding right atrioventricular state may be stored within a memory circuit of the implantable medical device. The information may be retrieved and the according left ventricular electrode automatically selected when the same right atrioventricular state is detected by the implantable medical device the next time.

In an embodiment of the present invention, more than one left ventricular electrode may be selected for pacing the left ventricle simultaneously in the aforementioned processes. That type of operating is known as multi-point pacing. For that purpose, more than one left ventricular electrode can be determined for pacing the left ventricle simultaneously, e.g. the physician may manually select more than one left ventricular electrode for a specific right atrioventricular state, and/or the implantable medical device is capable of performing aforementioned tests for suitable left ventricular electrodes using more than one left ventricular electrode simultaneously.

In a preferred embodiment, the control unit is adapted to discriminate at least two right atrioventricular conduction states, including a presence of intrinsic right atrioventricular conduction and an absence of right atrioventricular conduction.

It is further preferred, if the control unit is adapted to determine the presence or absence of an intrinsic right atrioventricular conduction depending on temporal condition based on an intrinsic or a stimulated right atrial event (As; Ap).

In particular, the control unit may be adapted to determine the presence of an intrinsic right atrioventricular conduction if a right ventricular sense-event (RVs) is recorded within a predetermined time period starting from an immediately preceding intrinsic or a stimulated right atrial event (As; Ap). Preferably, the duration of the predetermined time period depends on an actual heart rate or stimulation rate.

It is further preferred that the control unit is configured to enable a programmable sensitivity to the presented right-side atrioventricular conduction condition. In such a manner, the system could be configured to demand a threshold count of consecutive right side conduction sequences (of a common type) before administering changes to the LV paced vector. In other words, subject to this embodiment, a single emergent right-side conduction sequence alone might not prove sufficient for establishing the presence of a new atrioventricular conduction status. Under these conditions, the control unit would then only adapt LV vector support after a confirmed change in a patient's right-side conduction status. In particular, this capability is simply outlined as an extension for LV support and the clinician may deem it entirely acceptable, if not preferable to continually adapt the LV support subject to the right-side conduction conditions presented on each and every cardiac cycle.

Preferably, the control unit is adapted to determine the presence of an intrinsic right atrioventricular conduction if a number of recorded sensed right ventricular events exceeds a predetermined minimum, wherein each sensed right ventricular event within a specified count of cardiac cycles temporally correlates with a respective, preceding right atrial event (As; Ap). In this context, the recorded events do not necessarily need to occur in immediate succession, but may be part of several sequentially conducted period surveys of right side conduction. Furthermore, for determining the conditions for exceeding the predetermined minimum of sensed right ventricular events, a threshold could be defined for paced right ventricular events.

Preferably, the control unit according to the present invention is adapted to select that left ventricular electrode that corresponds to the longest conduction time to any of the left ventricular electrodes, starting from a right ventricular event.

With respect to all embodiments, it is preferred if the control unit is adapted to select the left ventricular electrode that corresponds to the longest conduction time to any of the left ventricular electrodes, starting from the right ventricular event. This longest delay condition may be uniquely aligned with the prevailing right-side conduction condition.

Preferably, the control unit according to the present invention is adapted to determine and/or select the left ventricular electrode that corresponds with the longest RV to LV conduction time emergent from a given type of right-side event activity.

In particular, it is preferred if the control unit is adapted
- to select that left ventricular electrode for left ventricular pacing that provides the longest conduction time between a right ventricular sensed event to a left ventricular sensed event, if right atrioventricular intrinsic conduction exists, and
- to select that left ventricular electrode for left ventricular pacing that provides the longest conduction time between a right ventricular paced event to a left ventricular sensed event, if no right atrioventricular intrinsic conduction exists.

In a preferred embodiment of the present invention, the control unit of the implantable medical device is adapted to
- select different left ventricular electrodes for different right atrioventricular conduction states,
- associate a selected left ventricular electrode to the corresponding right atrioventricular conduction state and
- to store the information on the association in a memory circuit.

According to a preferred embodiment of the present invention, The implantable medical device according to one of the preceding claims, wherein the implantable medical device is configured to determine right atrioventricular conduction state and select one of the plurality of electrode poles of the multipolar left ventricular stimulation electrode lead automatically in a periodical manner,
or
wherein determination of right atrioventricular conduction state and selection of one of the plurality of electrode poles of the multipolar left ventricular stimulation electrode lead is performed according to a manual process via receiving input from a user,
or
wherein determination of right atrioventricular conduction state and selection of one of the plurality of electrode poles of the multipolar left ventricular stimulation electrode lead is performed according to a combination of an automatic and manual process, wherein limits may be established by the clinician that restrict the available pool of selectable LV pacing vectors.

Preferably, the implantable medical device is configured to determine right atrioventricular conduction state on a beat-by-beat basis and, in turn, automatically select one of the plurality of electrode poles of the multipolar left ventricular stimulation electrode either in coordination with this beat-by-beat evaluation or subject to the recognition of a new threshold-dictated prevalence of a changed right-side conduction condition. The appropriate choices for the LV pacing vectors would, in automated implementations, ideally leverage a periodic check of the RV to LV conduction times during ambulatory states to update the best-case support options. This automatic adjustment of the LV pacing support would, in favorable embodiments, be influenced, and perhaps limited by restrictions imposed by the clinician at follow-up to avoid unintended consequences such as adoption of pacing vectors that would prove likely to detrimentally impact device services times. In non-automated formats, the clinician could employ follow-up tests to establish specific right-side atrioventricular conduction states and in turn evaluate the RV to LV conduction times as assessed across the the plurality of electrode poles offered by the multipolar left ventricular stimulation electrode.

In either case, the implant would maintain a specified prescription for the appropriate LV vector to be used for each right-side conduction condition and said prescription would either update during ambulatory states or via updates at follow-up. It is also possible that determination of right atrioventricular conduction state and selection of one of the plurality of electrode poles of the multipolar left ventricular stimulation electrode lead is performed according to a combination of an automatic and manual process. For instance, the automatic determination and selection processes may be subject to restrictions preset by the user, or the manual process may be supported by automatic sensing and evaluation algorithms and processes.

In a preferred embodiment of the present invention, the control unit of the implantable medical device is adapted to provide selection of more than one of the plurality of electrode poles of the multipolar left ventricular stimulation electrode lead. Such multi-point pacing support may further aid in establishing improved heart function in response to the determined right atrioventricular conduction state.

Further, with respect to all embodiments, it is preferred if the control unit is adapted to determine the presence or absence of an intrinsic right atrioventricular conduction on a beat-to-beat basis. In other words, the determination of the presence or absence of an intrinsic right atrioventricular conduction is performed in each heart cycle, e.g. coordinated with each sensed or paced right ventricular event.

Preferably, the control unit is connected to a quadripolar left ventricular electrode lead.

Preferably, the implantable medical device is a heart stimulator that is adapted to provide a cardiac resynchronization therapy (CRT).

### BRIEF DESCRIPTION OF THE DRAWINGS

The above and other aspects, features and advantages of the present invention will be more apparent from the following more particular description thereof, presented in conjunction with the following drawings wherein:
- Figure 1: shows a three chamber bi-ventricular implantable cardioverter/defibrillator (ICD).
- Figures 2: is a schematic diagram of an embodiment of the device modules of the ICD of Figure 1.
- Figure 3a: provides an example illustration where intrinsic right-side conduction instates a longest RV to LV conduction condition at the second (from top) left ventricular electrode pole.
- Figure 3b: provides an example illustration where a lack of intrinsic right-side conduction instates a longest RV to LV conduction condition at the topmost left ventricular electrode pole.
- Figure 4: illustrates inter-ventricular conduction times in case of an intact intrinsic right-side conduction.
- Figure 5: illustrates inter-ventricular conduction times in case of compromised intrinsic right-side conduction, involving a paced right ventricular event.
- Figures 6a to 6c: illustrate the effect of poor (a and b) and optimal (c) ventricular coordination.

The following description is of the best mode presently contemplated for carrying out the invention. This description is not to be taken in a limiting sense, but is made merely for the purpose of describing the general principles of the invention. The scope of the invention should be determined with reference to the claims.

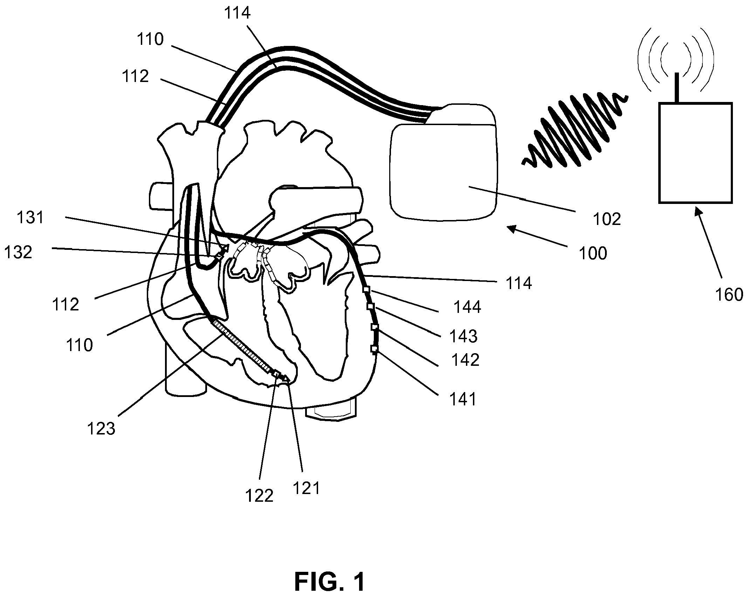

In Figure 1 the implantable medical device (also referred to as implantable cardiac device) is a three chamber biventricular pacemaker and cardioverter/defibrillator 100 that is connected to pacing/sensing leads 110, 112 and 114 placed in a heart is illustrated.

As shown in Figure 1, the preferred embodiment is to couple the disclosed technology with an implantable bi-ventricular defibrillator.

The implantable medical device 100 is electrically coupled to heart by way of leads 110, 112 and 114.

Lead 110 is a right ventricular electrode lead that has a pair of ventricular stimulation and sensing electrodes 121 and 122 that are in contact with the right ventricle of heart. In case the implantable medical device includes a defibrillation function, a ventricular defibrillation shock coil 123 is arranged on lead 110.

Lead 112 is a right atrial electrode lead that has a pair of right atrial electrodes 131 and 132 that are in contact with the right atria of the heart.

Electrodes 121 and 131 are tip electrodes at the very distal end of leads 110 and 112, respectively. Electrode 131 is a right atrial tip electrode RA Tip and electrode 121 is a right ventricular tip electrode. Electrodes 122 and 132 are ring electrodes in close proximity but electrically isolated from the respective tip electrodes 121 and 131. Electrode 132 forms a right atrial ring electrode RA Ring and electrode 122 forms a right ventricular ring electrode RV Ring. Ventricular defibrillation shock coil 123 is a coil electrode providing a relatively large geometric area when compared to the stimulation electrodes 121, 122, 131 and 132.

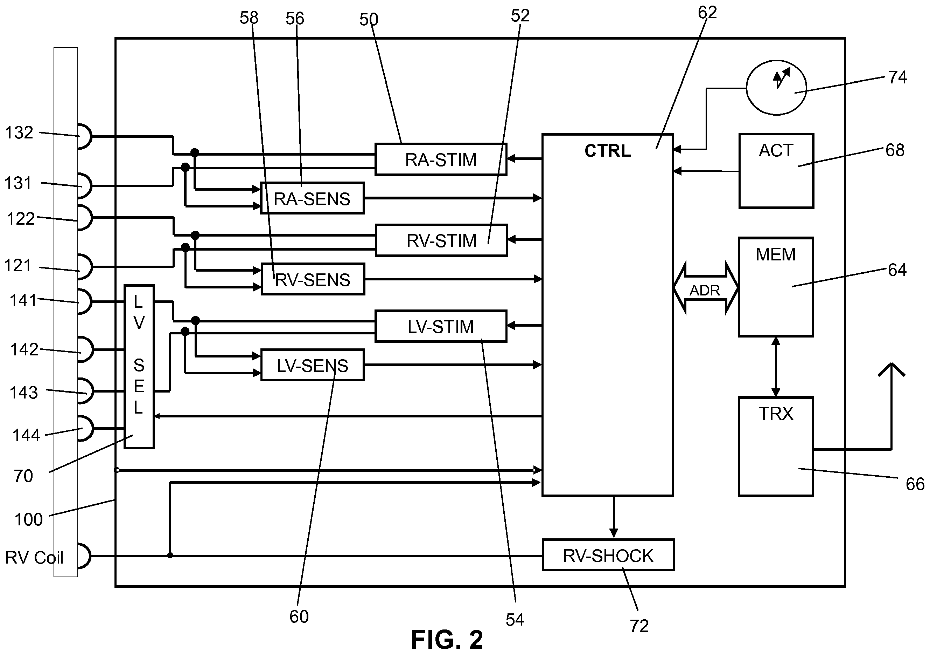

Lead 114 is a left ventricular electrode lead passing through the coronary sinus of heart and having a left ventricular tip electrode LV TIP 141 and left ventricular ring electrodes LV RING 142, 143 and 144. Further, a left ventricular defibrillation shock coil (not shown) may be arranged on lead 114. It is noted that the number of left ventricular ring electrodes may vary depending on the electrode lead that is used. In the context of Figures 1 and 2, one left ventricular ring electrode LV-RING is referred to the acts as pars pro toto.

Implantable medical device 100 has a case 102 made from electrically conductive material such as titanium that can serve as a large surface electrode.

The plurality of left ventricular electrodes 141, 142, 143 and 144 allow for a number of different pacing sites for stimulating the left ventricle.

Figure 2 illustrates a simplified block diagram of an implantable medical device 100. During operation of the implantable medical device 100, electrode leads 110, 112 and 114 are connected to respective output/input terminals of the implantable medical device 100 as indicated in Figure 2 and carry stimulating pulses to the tip electrodes 121, 131 and one of 141, 142, 143 and 144 from a right atrial stimulation pulse generator RA-STIM 50, a right ventricular pulse generator RV-STIM 52 and a left ventricular pulse generator LV-STIM 54, respectively. Selection of one of left ventricular electrode poles 141, 142, 143 and 144 as the left ventricular stimulation electrode pole is done by a control unit 62 via selection unit 70. Control unit 62 selects the left ventricular stimulation electrode based on a respectively determined right atrioventricular conduction state, i.e. whether there is intrinsic right ventricular conduction or not.

For assessing conduction from the right atrium to the right ventricle of the heart in the context of the present invention, the implantable medical device 100 requires atrial and/or ventricular sensing support and capacity. Such capacity include electric components (as e.g. aforementioned right atrial electrode lead 112 which is in direct contact with the heart tissue in the right atrium, or a lead having an electrode for sensing in the right atrium without direct contact with the heart tissue, as for example a right side ventricular lead having a floating electrode which is located in the atrium) as well as respective software units for sensing, processing and evaluation of acquired signals.

Further, electrical signals from the right atrium are carried from the electrode pair 131 and 132, through the lead 112, to the input terminal of a right atrial channel sensing stage RA-SENS 56; and electrical signals from the right ventricle are carried from the electrode pair 121 and 122, through the lead 110, to the input terminal of a right ventricular sensing stage RV-SENS 58. Likewise electrical signals from the left ventricle are carried from the electrode pairs on lead 114, to the input terminal of a left ventricular sensing stage LV-SENS 60.

A defibrillation shock generator RV-SHOCK 72 is connected to the shock coil 123 via right ventricular electrode lead 110.

Controlling the implantable medical device 100 is a control unit CTRL 62 that is connected to sensing stages RA-SENS 56, RV-SENS 58 and LV-SENS 60, to stimulation pulse generators RA-STIM 50, RV-STIM 52 and LV-STIM 54, to defibrillation shock generator RV-SHOCK 72 and to selection unit LV SEL 70. Control unit CTRL 62 receives the output signals from the atrial sensing stage RA-SENS 56, from the right ventricular sensing stage RV-SENS 58 and from the left ventricular sensing stage LV-SENS 60. The output signals of sensing stages RA-SENS 56, RV-SENS 58 and LV-SENS 60 are generated each time that a P-wave representing an intrinsic atrial event or an R-wave representing an intrinsic ventricular event, respectively, is sensed within the heart. An As-signal is generated, when the atrial sensing stage RA-SENS 56 detects a P-wave and a RVs-signal is generated, when the right ventricular sensing stage RV-SENS 58 detects an R-wave.

In general, control unit 62 is preferably adapted to monitor right atrioventricular conduction behavior, in particular the time sequence of atrial events (As or Ap) and the next ventricular event in order to determine whether there is an intrinsic right-ventricular following an atrial event within a certain time.

When control unit 62 receives a RVs-signal within a predetermined time period starting from an As-signal or an Ap event, control unit 62 switches in an operation mode for intact intrinsic atrioventricular (AV) conduction and delivery of a right ventricular pacing pulse is inhibited. Otherwise, control unit 62 switches in an operation mode corresponding to a lack of intrinsic atrioventricular (AV) conduction. As a consequence a right ventricular pacing pulse may be delivered and the pacing site for the left ventricle may be altered by selecting a different left ventricular electrode for delivery of the left ventricular pacing pulse than in the operation mode for intact intrinsic atrioventricular (AV) conduction.

Control unit CTRL 62 also generates stimulation signals that are sent to the atrial stimulation pulse generator RA-STIM 50, the right ventricular stimulation pulse generator RV-STIM 52 and the left ventricular stimulation pulse generator LV-STIM 54, respectively. These stimulation signals are generated each time that a stimulation pulse is to be generated by the respective pulse generator RA-STIM 50, RV-STIM 52 or LV-STIM 54. The atrial stimulation signal is referred to simply as the "RA-pulse", and the ventricular stimulation signal is referred to as the "RV-pulse" or the "LV-pulse", respectively. During the time that either an atrial stimulation pulse or ventricular stimulation pulse is being delivered to the heart, the corresponding sensing stage, RA-SENS 56, RV-SENS 58 and/or LV-SENS 60, is typically disabled by way of a blanking signal presented to these amplifiers from the control unit CTRL 62, respectively. This blanking action prevents the sensing stages RA-SENS 56, RV-SENS 58 and LV-SENS 60 from becoming saturated from the relatively large stimulation pulses that are present at their input terminals during this time. This blanking action also helps prevent residual electrical signals present in the muscle tissue as a result of a stimulation pulse delivered from implantable medical device 100 from being interpreted as P-waves or R-waves.

Control unit CTRL 62 comprises circuitry for timing ventricular and/or atrial stimulation pulses according to an adequate stimulation rate that can be adapted to a patient's hemodynamic need as pointed out below. Timing is assisted by clock 74 connected to control unit 62.

Still referring to Figure 2, the implantable medical device 100 includes a memory circuit MEM 64 that is coupled to the control unit CTRL 62 over a suitable data/address bus ADR. This memory circuit MEM 64 allows certain control parameters, used by the control unit CTRL 62 in controlling the operation of the implantable medical device 100, to be programmably stored and modified, as required, in order to customize the implantable medical device's operation to suit the needs of a particular patient. Such data includes the basic timing intervals used during operation of the implantable medical device 100 and AV delay values and hysteresis AV delay values in particular.

Further, data sensed during the operation of the implantable medical device 100 may be stored in the memory MEM 64 for later retrieval and analysis.

A telemetry circuit TRX 66 is further included in the implantable medical device 100. This telemetry circuit TRX 66 is connected to the control unit CTRL 62 by way of a suitable command/data bus. Telemetry circuit TRX 66 allows for wireless data exchange between the implantable medical device 100 and some remote programming or analyzing device which can be part of a centralized service center serving multiple implantable medical devices. Telemetry circuit TRX 66 allows data communication with an external device 160 (see Figure 1).

In Figure 2, . those portions of the implantable medical device 100 that interface with the right atrium, e.g., the lead 112, the P-wave sensing stage RA-SENS 56, the atrial stimulation pulse generator RA-STIM 50 and corresponding portions of the control unit CTRL 62, are commonly referred to as the atrial channel. Similarly, those portions of the implantable medical device 100 that interface with the right ventricle, e.g., the lead 110, the R-wave sensing stage RV-SENS 58, the ventricular stimulation pulse generator RV-STIM 52, and corresponding portions of the control unit CTRL 62, are commonly referred to as the right ventricular channel. Accordingly, the implantable medical device may include a left ventricular lead having electrodes with sensing and/or pacing functionalities. Respectively, those portions of the implantable medical device 100 that are coupled to signals from the left ventricle and corresponding portions of the control unit CTRL 62, can be referred to as the left ventricular channel.

In order to be able to detect periods of physical activity of a patient indicating that the patient is awake and in order to allow rate adaptive pacing in a DDDR or a DDIR mode, for example, the implantable medical device 100 further includes a physiological sensor ACT 68 that is connected to the control unit CTRL 62 of the implantable medical device 100. While this sensor ACT 68 is illustrated in Figure 2 as being included within the implantable medical device 100, it is to be understood that the sensor may also be external to the implantable medical device 100, yet still be implanted within or carried by the patient. A common type of sensor is an accelerometer, such as an inertial proof mass mounted within the implantable medical device. Other types of physiologic sensors are also known, such as sensors that sense the oxygen content of blood, respiration rate, blood pH, intracardiac impedance changes, and the like. The type of sensor used is not critical to the present invention. Any sensor capable of sensing some physiological parameter relatable to physical activity of a patient can be used. Such sensors are commonly used with "rate-responsive" implantable medical devices in order to adjust the rate of the implantable medical device in a manner that tracks the physiological needs of the patient. The output of sensor 68 represents an activity level.

By means of the output signal of activity sensor 68 the control unit 62 is able to assign each intrinsic heart rate to an activity thus enabling collection of intrinsic heart rate value for a patient's state of rest and a patient's state of exercise separately.

The control unit CTRL 62 is adapted to determine an adequate heart rate or stimulation rate in any manner known as such.

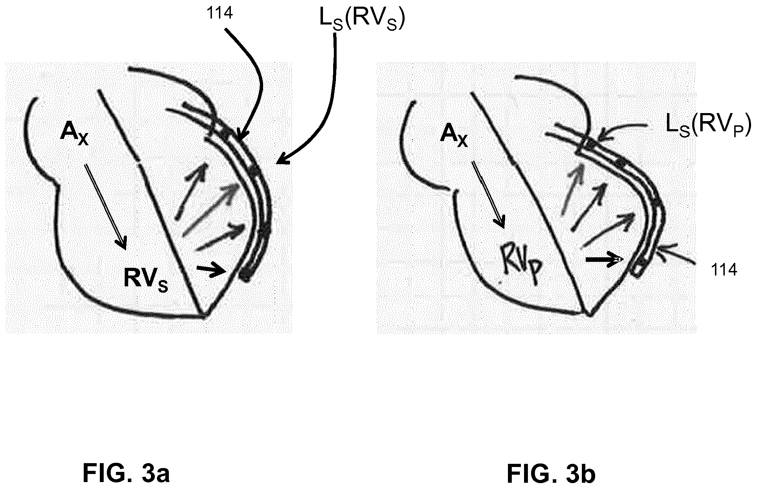

Figures 3a and 3b illustrate that typically the inter-ventricular conduction time from the right ventricle to a particular site in the left ventricle (position of a respective one of a plurality of electrode poles 141, 142, 143 and 144 on the left ventricular electrode lead 114) depends on the origin of the right ventricular activity. Figure 3a illustrates an example configuration wherein an intrinsic right ventricular event is last sensed by the second (from top) left ventricular electrode pole 143. Alternatively, Figure 3b provides an example configuration wherein a paced (stimulated) right ventricular event is last sensed by the topmost left ventricular electrode pole 144.

In Figure 3a the latest conducted left ventricular event that corresponds to the maximum sensed inter-ventricular propagation delay (RVs to LVs) is marked with L_{S}(RV_{S}). In Fig. 3b, the latest conducted left ventricular event in case of a paced right ventricular event (corresponding to maximum paced inter-ventricular propagation delay (RVp to LVs)) is marked with L_{S}(RV_{P}). As can be taken from Figures 3a and 3b the latest activation sites in the left ventricle are not the same and are based upon the presence or absence of intrinsic right atrial ventricular conduction.

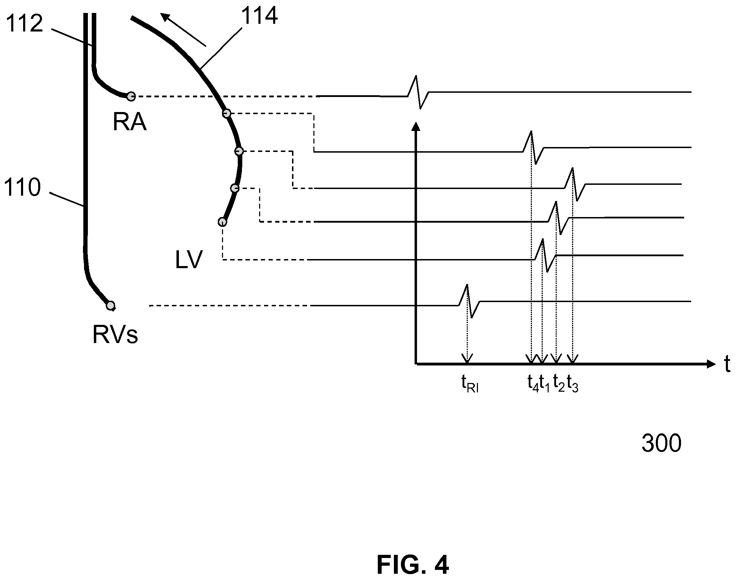

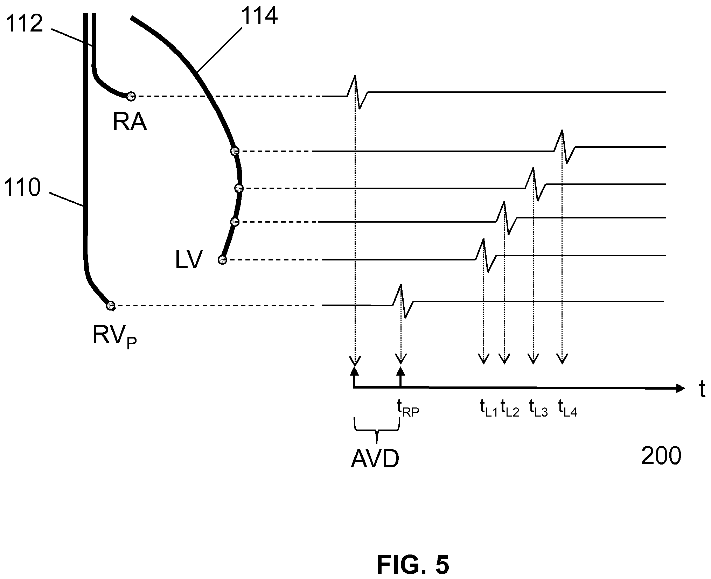

Figure 4 discloses inter-ventricular conduction times in case of an intact right-side intrinsic atrioventricular conduction while Figure 5 discloses inter-ventricular conduction times in case of a compromised intrinsic right-side atrioventricular conduction. Control unit 62 preferably is adapted to determine the longest inter-ventricular conduction time for each class of right-side conduction status, that is, depending on whether or not intrinsic right atrioventricular conduction is present (as in Figure 4) or not (as in Figure 5). Control unit 62 can do so by measuring times t_{RI}, t₁, t₂, t₃ and t₄ for each class of right-side conduction.

In the examples given in Figures 3a, 3b, 4 and 5, control unit 62 would determine that left ventricular ring electrode 143 would provide the best left ventricular pacing site in case of a presence of intrinsic right atrioventricular conduction, while left ventricular ring electrode 144 would provide the best left ventricular pacing site in case of an absence of intrinsic right atrioventricular conduction.

The main purpose of the sensing stages 56, 58 and 60 is to detect a natural (intrinsic) contraction of the respective heart chamber in order to generate a sense event signal like an atrial sense event As, a right ventricular sense event RVs and a left ventricular sense event LVs. These sense events are processed by the control unit CTRL 62 in order to inhibit a delivery of a stimulation pulse when the implantable medical device is operating in a demand mode or in order to determine a time interval between an atrial event and a point of time. Another type of event to be processed by the control unit CTRL 62 would be the delivery of a stimulation pulse to a respective heart chamber. Delivery of a stimulation pulse causes a paced event such as an atrial paced event Ap, a right ventricular paced event RVp and a left ventricular paced event LVp.

According to a preferred embodiment of the invention, different left ventricular pacing sites are chosen depending on whether control unit 62 receives an RVs signal appropriately coordinated within a preceding Ax and thus detects an intrinsic atrioventricular conduction condition or not. Different pacing sited are established by choosing one (or a sub-set) of left ventricular electrodes 141, 142, 143 or 144 for delivery of left ventricular stimulation pulses.

Left ventricular pacing is typically deemed optimal when it is administered at the latest activated region within the left ventricle. Determining the latest activated region has historically been assessed only in the context of intrinsic activation without explicit consideration for latest site locations emergent from paced right ventricular activity. In the context of CRT therapies, the patient often witnesses pacing in both ventricles and, as such, targeting the left ventricular pacing site to the latest activated region emergent from right atrioventricular conducted events is likely an inappropriate choice. Such conclusions emerge from the reality that intrinsic right ventricular activity and paced right ventricular activity often change the physical location of the latest activated region within the left ventricle.

Therefore, control unit 62 is configured for monitoring of right atrioventricular conduction behaviors as a switch for left ventricular pacing vector assignment. In cases where intrinsic right atrioventricular conduction exists, the left ventricular pacing vector should be the one affiliated with the longest intrinsic inter-ventricular (RVs to LVs) conduction path and in cases where intrinsic right atrioventricular A to RV conduction does not exist, the left ventricular pacing vector should be the one affiliated with the longest paced inter-ventricular (RVp to LVs) conduction path.

Such a capability makes use of a quadri-polar left ventricular electrode lead 114. Such leads provide the implantable medical device 100 with physical means for assessing inter-ventricular (RV to LV) conduction times across a multitude of distinct left ventricular pacing sites and, in turn, provisioning the selection of a pacing vector in accordance with the latest activated region subject to either sensed right ventricular events (RVs) (graded with respect to their coordination with preceding atrial events) or paced right ventricular events (RVp). Pairing such a quadri-polar left ventricular electrode lead 114 with an in-implant algorithm that either continually or periodically monitors right-side atrioventricular conduction would facilitate the ability to use the presence or absence of right atrioventricular conduction as the pacing vector determinant.

During normal biventricular (BiV) modes, pacing typically occurs in either 1.) both ventricles (what is referred to as BiV pacing), 2.) only in the left ventricle as an event coordinated with sensed right ventricular activity, or 3.) only in the right ventricle (wherein 2.) and 3.) t are referred to as CRT pacing). The feature proposed herein can easily be combined with normal BiV modes through the continual monitoring of right atrioventricular conduction behaviors. Any time a conducted right atrioventricular (A to RVs) event sequence occurs, the left ventricular vector associated with maximum inter-ventricular (RVs to LVs) propagation delays is used and in cases where no conducted event arrived in the right ventricle, the left ventricular vector associated with maximum paced right atrioventricular (RVp to LVs) propagation delays is used. Such an implementation preferably assesses and potentially modifies the left ventricular pacing vector on a beat-by-beat basis. A simplified approach may instead use a periodic RVp suppression survey (a capability we already have in our commercially-available CRT implants, i.e. the inhibition of right ventricular stimulation pulses in a demand mode) to assess for the presence of right-side conducted activity and avoid the continuous overhead associated with monitoring the event sequences on a beat-by-beat basis. In this context, it is noted that there are two periodic options available, one for assessing the right side conduction condition and a second for evaluating the best LV pacing support vector in implementations that provide ambulatory adaptation capabilities.

In automated modes that determine the appropriate LV pacing sites based on right-side conduction status, a periodic survey of RV to LV conduction values would occur. This capability would amount to an automated, implant-executed LVQC conduction algorithm that actively seeks the longest conduction sites within the LV as is illustrated in Figures 4 and 5. Within this routine the implant would be configured to measure the RV to LV conduction times at each pole of the LV pacing lead subject to conditions where right-side conduction prevailed and also subject to conditions where right-side conduction was absent. One could readily envision an elongation of the programmed AV delay facilitating the measurements for cases where right-side conduction prevailed and conversely employing an overdriven RV pacing routine (with shortened AV delays) as a means for collecting intraventricular conduction measurements in the absence of right-side AV conduction. The collected measurements could then be used to determine the appropriate LV pacing vector for each right-side conduction condition. In sophisticated implementations the selection of the LV vector could furthermore be influenced by restrictions imposed by the clinician at follow-up such as those that restricted vector selections that could instate problematic service lifetime implications. In simplified formats, establishing the appropriate LV vector could be limited to test-based data collection efforts coordinated at follow-up. The established vectors could then be held fixed within the implant until changes were made through subsequent follow-up test interactions.

A further simplification still may embody a method for tying the selection of a left ventricular stimulation electrode pole to mode selection. BiV modes could then employ the pacing site (left ventricular electrode pole selection) associated with paced inter-ventricular (RVp to LVs) maximum propagation delays and all LV-only BiV modes would leverage the pacing site (left ventricular electrode pole selection) associated with intrinsic inter-ventricular (RVs to LVs) maximum propagation delays.

The best implementation of this invention embodies a continual adaptation of the pacing vector to right-side conduction status on a beat-by-beat basis. Such a strategy ensures that each CRT supported cardiac cycle is paced from an optimal left ventricular site whether the cycle happened to present conducted right ventricular activity or not.

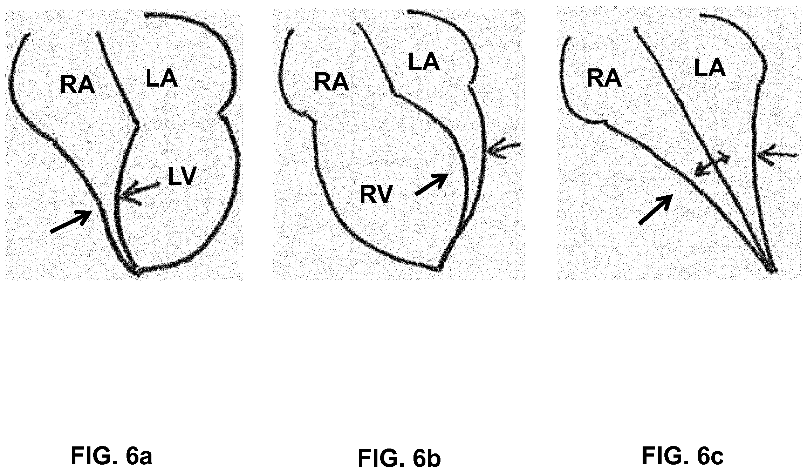

Figures 6a to 6c illustrate the effect of bad or good ventricular coordination, respectively. Figures 6a and 6b illustrate cases where the right ventricular and the left ventricular contractions are not well coordinated and thus the septal wall moves away from the central plane in the non-physiologic fashion during ventricular contraction activities. In the example illustrated Figure 6a the left ventricular contraction occurs too early with respect to the right ventricular contraction while in the example depicted in Figure 6b the right ventricular contraction occurs too early with respect to the left ventricular contraction.

Ideally, the right ventricular and the left ventricular conductions are well coordinated so that the septal wall maintains a fixed, centered position; see Figure 6c.

The implantable medical device 100 may further be configured for selective manual enablement or disablement of the automatic left ventricular pacing site selection altogether to thus provide a clinician with the freedom to choose whether or not to administer this adapted pacing site capability at all. Such freedom would afford maximal end-user discretion with regard to this feature's use/implementation. The specific pacing vectors used in each condition can either be clinician determined and selected at follow-up (likely as an offshoot of the LVQC test) or stem from an automated implant-administered survey of inter-ventricular conduction times. In turn, the feature can dynamically update the pacing vector based upon 1.) need (A to RV conduction present?) and 2.) inter-ventricular conduction times (either Max[RVs to LVs] or Max[RVp to LVs], respectively).

According to this invention, implantable medical devices offering CRT can dynamically update the pacing site location in response to emergent patient conduction behaviors. Such coordination can occur even in ambulatory conditions outside of clinical settings, in ways that avoid unwanted lag between device output and patient demand. This proposed adaptation of left ventricular pacing site locations additionally facilitates device/patient interactions that better mimic intrinsic, physiological septal wall motions and as such promote opportunities for substrate remodeling beyond what present CRT therapies can provide.

## Claims

1. An implantable medical device comprising a control unit, at least a right ventricular sensing unit and/or a right atrial sensing unit, wherein the right ventricular sensing unit is connected, or can be connected, to a right ventricular stimulation electrode lead having at least one electrode pole, and a stimulation unit that is connected, or can be connected, to a left ventricular stimulation electrode lead having a plurality of electrode poles, wherein the control unit is adapted to determine right-side atrioventricular conduction state and to provide selection of one of the plurality of electrode poles of the multipolar left ventricular stimulation electrode lead on the basis of the right-side atrioventricular conduction state.

2. The implantable medical device of claim 1, wherein for determining right side atrio-ventricular conduction state, the control unit is configured to determine whether a right ventricular sense-event (RVs) is present or not.

3. The implantable medical device according to at least one of the claims 1 or 2, wherein the control unit (62) is adapted to discriminate between at least two right-side atrioventricular conduction states, including a presence of intrinsic right-side atrioventricular conduction and/or an absence of right-side atrioventricular conduction and/or a disturbed right-side atrioventricular conduction state.

4. The implantable medical device according to at least one of the claims 2 or 3, wherein the control unit (62) is adapted to determine the presence or absence of an intrinsic right-side atrioventricular conduction depending on a temporal condition based on right-side ventricular activity with a preceding intrinsic or stimulated right atrial event (As; Ap).

5. The implantable medical device of claim 4, wherein the control unit (62) is adapted to determine the presence of an intrinsic right-side atrioventricular conduction if a right ventricular sense-event (RVs) is recorded within a predetermined time period starting from an immediately preceding intrinsic or a stimulated right atrial event (As; Ap).

6. The implantable medical device of claim 5, wherein the duration of the predetermined time period depends on an actual heart rate or stimulation rate.

7. The implantable medical device according to at least one of claims 4 to 6, wherein the control unit (62) is adapted to determine the presence of an intrinsic right atrioventricular conduction if a number of recorded sensed right ventricular events exceeds a predetermined minimum, wherein each sensed right ventricular event of the number of recorded sensed right ventricular events relates to a respective right atrial event (As; Ap) of sequence of immediately consecutive atrial events.

8. The implantable medical device according to at least one of claims 1 to 7, wherein the control unit (62) is adapted to determine and/or select the left ventricular electrode that corresponds with the longest conduction time to any of the left ventricular electrodes, starting from a right ventricular event.

9. The implantable medical device according to one of the preceding claims, wherein the control unit (62) is adapted to
- select different left ventricular electrodes for different right atrioventricular conduction states,
- associate a selected left ventricular electrode to the corresponding right atrioventricular conduction state and
- to store the information on the association in a memory circuit.

10. The implantable medical device according to one of the preceding claims, wherein the implantable medical device is configured to determine right atrioventricular conduction state and select of one of the plurality of electrode poles of the multipolar left ventricular stimulation electrode lead automatically in a periodical manner,
or
wherein determination of right atrioventricular conduction state and selection of one of the plurality of electrode poles of the multipolar left ventricular stimulation electrode lead is performed according to a manual process via receiving input from a user,
or
wherein determination of right atrioventricular conduction state and selection of one of the plurality of electrode poles of the multipolar left ventricular stimulation electrode lead is performed according to a combination of an automatic and manual process.

11. The implantable medical device according to one of the preceding claims, wherein the control unit (62) is adapted to cause selection of more than one of the plurality of electrode poles of the multipolar left ventricular stimulation electrode lead depending on a respective determined right-side atrioventricular conduction state.

12. The implantable medical device of at least one of the claims 2 to 11, wherein the control unit (62) is adapted
- to select that left ventricular electrode for left ventricular pacing that provides the longest conduction time between a right ventricular sensed event to a left ventricular sensed event, if right atrioventricular intrinsic conduction exists, and
- to select that left ventricular electrode for left ventricular pacing that provides the longest conduction time between a right ventricular paced event to a left ventricular sensed event, if no right atrioventricular intrinsic conduction exists.

13. The implantable medical device according to at least one of claims 1 to 12, wherein the control unit (62) is adapted to determine the presence or absence of an intrinsic right atrioventricular conduction on a beat-to-beat basis.

14. The implantable medical device according to at least one of claims 1 to 13, wherein the control unit is connected to a quadri-polar left ventricular electrode lead (114).

15. The implantable medical device according to at least one of claims 1 to 14, wherein the implantable medical device is a heart stimulator that is adapted to provide a cardiac resynchronization therapy.
